# EUROPEAN PATENT APPLICATION

(11) **EP 0 765 927 A1**
(43) Date of publication of application: **02.04.1997**
(21) Application number: 96306921.6
(22) Date of filing: 24.09.1996
(51) Int. Cl.: C10G 47/00, C07C 4/18

(54) **Method for producing beta-methylnaphthalene**

(30) Priority: 27.09.1995 JP 273458/95
(71) Applicant: FUJI OIL COMPANY, LIMITED, Tokyo (JP); PETROLEUM ENERGY CENTER, Minato-ku, Tokyo (JP)
(72) Inventor: Tashiro, Masaharu, Yokohama-shi, Kanagawa 247 (JP); Tsutsui, Toshio, Yokohama-shi, Kanagawa 244 (JP); Kubota, Osamu, Sodegaura-shi, Chiba 299-02 (JP); Okada, Shinichi, Sodegaura-shi, Chiba 299-02 (JP); Nakamura, Tosihito, Sodegaura-shi, Chiba 299-02 (JP)
(74) Representative: West, Alan Harry

(57) **Abstract**

Chemical grade β-methylnaphthalene of purity adequate for industrial use is produced in bulk at low cost by hydrodealkylation of a feed oil containing an alkyl naphthalene having at least two methyl groups, for example light cycle oil, in the presence of a catalyst having at least one metal species selected from vanadium, chromium, nickel, rhodium, platinum, iridium, and compounds thereof as an active component and a carrier therefor containing at least one of alumina and silica as its primary component, under a hydrogen partial pressure 90 to 5,000 kPa, at a temperature of 450 and 650°C, and for a contact time of 3 to 35 seconds.

## Description

The present invention relates to a method for producing β-methylnaphthalene by catalytic hydrodealkylation and more particularly to a method for producing chemical grade β-methylnaphthalene in a high yield from a feed oil containing an alkyl naphthalene having at least two methyl groups.

β-Methylnaphthalene has a boiling point of 241.1°C and a melting point of 34.4°C. At room temperature, it is a crystalline colourless solid. It has conventionally been obtained by crystallization from coal tar fractions boiling at around 240°C. It has also been produced by vapour phase methylation of naphthalene with methyl chloride, and by reduction of 2-methyl-1,4-naphthoquinone.

Recently β-methylnaphthalene has become more valuable as a starting material for the commercial production of polyesters. However, the conventional methods for producing β-methylnaphthalene cannot supply it in sufficient amounts to meet demand. Moreover, β-methylnaphthalene prepared by such conventional methods is insufficiently pure for industrial use.

It is therefore technically and economically impractical to implement mass-production of chemical grade β-methylnaphthalene, i.e., β-methylnaphthalene having a purity of not less than 97-98% and containing minor amounts of such non-hydrocarbon impurities as nitrogen and sulphur, by conventional means. A new process applicable to the industrial production of β-methylnaphthalene is therefore necessary to ensure its adequate supply at low cost.

Since β-methylnaphthalene was not previously in great demand as an industrial starting material, attempts to produce it industrially have not hitherto attracted much attention. As a result, no attempts other than those mentioned above had been made for developing a new method for producing chemical grade β-methylnaphthalene.

The present inventors have searched for an inexpensive feed oil which is readily available in the petroleum or petro-chemical industries as a replacement for the conventional coal tar and have noted an intermediate product obtained in various crude oil refining processes particularly the intermediate fraction having a high content of naphthalene compounds which is generated in the catalytic-cracking of heavy fractions of a crude oil to produce a gasoline stock.

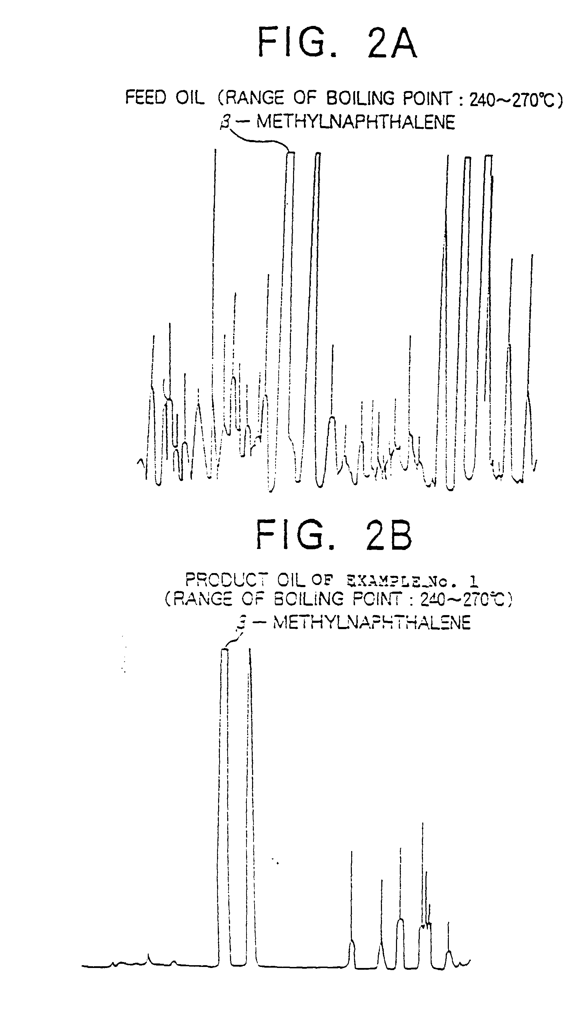

Attempts to separate and collect β-methylnaphthalene directly from that intermediate fraction led them to conclude that it was technically difficult and economically impracticable by this route to obtain high grade β-methylnaphthalene having high purity and containing minor amounts of non-hydrocarbon compounds such as sulphur-containing compounds and nitrogen-containing compounds, i.e. so-called chemical grade β-methylnaphthalene. This is because first, such an intermediate fraction contains too small a ratio of β-methylnaphthalene against total naphthalene compounds, and second, as shown in the gas chromatogram of Fig. 2A, the intermediate fraction contains not only β-methylnaphthalene but also significant amounts of a variety of hydrocarbons having boiling points close to the boiling point of β-methylnaphthalene, as well as non-hydrocarbon impurities such as sulphur-containing compounds and nitrogen-containing compounds.

Further research has revealed that β-methylnaphthalene may be effectively mass-produced with a significant reduction in the amounts of impurities by catalytic hydrodealkylating a feed oil containing alkyl naphthalenes such as the above-mentioned intermediate fraction. It has been found that this economically advantageous and attractive approach enables the conversion of such a feed into a product having an increased β-methylnaphthalene content and having distillation properties that allow easy separation of high grade β-methylnaphthalene.

Examination of known hydrodealkylation processes for alkyl aromatic compounds, such as those described in Japanese Laid-Open Patent Application Nos. 206686/1990, 298347/1980 and 304033/1990, to evaluate their applicability to β-methylnaphthalene production, reveals that they are not applicable to the production of β-methylnaphthalenes having a specific methyl group, particularly β-methylnaphthalene, although these methods are capable of producing naphthalenes by complete dealkylation.

The process described in Japanese Laid-Open Patent Application No. 206686/1990 (US Patent No. 5,132,480) for the catalytic hydrodealkylation of a feed oil containing alkylaromatic hydrocarbons using a two-column apparatus, one column for the catalytic hydrodealkylation and the other for catalyst regeneration, involves hydrodealkylation of the feed oil in a fluidized bed of a catalyst comprising substantially spherical particles having a weight mean diameter of 25 to 250 µm, an apparent density of 0.3 to 1.5g/cm³, a pore volume of 0.10 to 1.5 cm³/g under a total pressure of 195 to 2950 kPa, a hydrogen partial pressure of 145 to 1950 kPa and a temperature of 350 to 700°C.

The process described in Japanese Laid-Open Patent Application No. 298347/1990 (US Patent No. 5,053,574) for the catalytic hydrodealkylation of alkylaromatic hydrocarbons comprises contacting an alkylaromatic compound under a hydrogen partial pressure of 98 to 4900 kPa and at a temperature of 450 to 700°C with a catalyst which comprises porous alumina particles with coke deposited in the pores, the alumina particles having a pore volume of 0.1 to 1.5 cm³/g and a specific surface area of 5 to 500 m²/g, the quantity of coke being 1 to 30% by weight of the alumina particles, and the pore volume and the specific surface area of the catalyst being 0.05 to 1.5 cm³/g and 1 to 500 m²/g, respectively.

Japanese Laid-Open Patent Application No. 304033/1990, referred to above, describes the catalytic hydrodealkylation of alkylaromatic hydrocarbons at a temperature of 450 to 680°C under hydrogen with a catalyst which comprises vanadium (V) carried by alumina particles.

Despite these difficulties in conventional approaches to the production of chemical grade β-methylnaphthalene the present inventors have developed a method for the industrial production of chemical grade β-methylnaphthalene using, as a feed, a light cycle oil (hereinafter referred to as LCO) containing dimethylnaphthalene and trimethylnaphthalene under specific reaction conditions to achieve hydrodealkylation of the feed oil, in the presence of a known catalyst.

LCO is a fraction having a boiling point range of 170-370°C, obtained as a byproduct of fluidized catalytic cracking of heavy fractions of crude oil to produce a gasoline stock. LCO contains a relatively large amount of alkyl naphthalenes of a wide variety. Preferably, the light cycle oil or its equivalent used as the feed oil has a boiling point range of 200 to 320°C, and more preferably 230 to 300°C.

Accordingly, the present invention provides a method for producing β-methylnaphthalene which comprises hydrodealkylating a feed oil containing an alkyl naphthalene having at least two methyl groups in the presence of a catalyst having at least one metal species selected from vanadium (V), chromium (Cr), nickel (Ni), rhodium (Rh) , platinum (Pt), iridium (Ir), and compounds thereof as an active component and a carrier therefor containing at least one of alumina and silica as its primary component, under a hydrogen partial pressure 90 to 5,000 kPa, at a temperature of 450 and 650°C, and for a contact time of 3 to 35 seconds.

Using this method it is possible to obtain β-methylnaphthalene in a high yield and in sufficient purity to be used as an industrial starting material from a source oil that is both inexpensive and readily available as a product of the petroleum and petro-chemical industries.

There is no particular limitation placed on the feed oil used in the method of the present invention, so long as it contains an alkyl naphthalene having at least two methyl groups, e.g. dimethylnaphthalene and/or trimethylnaphthalene. In order to produce β-methylnaphthalene with a high yield, it is preferred that the feed oil contains not less than 10 weight % of at least either dimethylnaphthalene or trimethylnaphthalene. Examples of preferred oils include byproduct oils, such as light cycle oil (LCO) and heavy cycle oil (HCO), of the catalytic cracking of heavy fractions of crude oil, product oils of the catalytic reforming of light fractions of crude oil, byproduct oils of naphtha cracking, coal tar, and liquified coal oils.

These feed oils may contain sulphur-containing compounds such as benzothiophene, nitrogen-containing compounds such as quinolines and indoles, and oxygen-containing compounds such as phenols, benzofurans and dibenzofurans.

In the method of the present invention, the alkyl naphthalenes contained in the feed oil, which undergoing hydrodealkylation, are selectively converted into lower alkyl naphthalenes having a reduced number of alkyl groups as compared with the original alkyl naphthalenes. Also, part of any α-methylnaphthalene is converted into β-methylnaphthalene through isomerization.

The catalyst used in the method of the present invention may be known or commercially available so long as it includes at least one metal species selected from vanadium (V), chromium (Cr), nickel (Ni), rhodium (Rh), platinum (Pt), iridium (Ir), and compounds of these metals as an active component, and a carrier therefor containing at least one of alumina and silica as its primary component. Compounds of these metals include the oxides and sulphides. Even when the catalyst further contains a small amount of molybdenum (Mo), rhenium (Re) and other trace metals or compounds, the catalytic activity is not adversely affected. The catalyst may also contain phosphorus (P) and other trace non-metal elements as well as various oxides, such as oxides of barium (Ba), lanthanum (La), potassium (K) and calcium (Ca), including titania and magnesia.

For an alumina carrier containing alumina as the primary component for example, it is preferred that approximately 10 weight % of silica is contained in the carrier to increase the thermal stability of the catalyst particles.

The reactor for carrying out the method of the invention may be of a fixed bed, a moving bed, or a fluidized bed type. Alternatively, the reactor may have a catalyst bed of a type other than the above.

Use of a fluidized bed reactor is preferred, first because it achieves enhanced contact efficiency between the feed oil and the catalyst, second because it permits a degree of control and flexibility of the reaction conditions, including the contact time and the reactor temperature, and third because it allows the reaction conditions be kept stable and uniform throughout the catalyst bed due to the particle mixing effect unique to a fluidized bed, even during an exothermic reaction such as hydrodealkylation.

When a fluidized bed is used, the catalyst preferably takes the form of substantially spherical particles having a weight-mean diameter between 25 and 250 µm, and more preferably between 40 and 120 µm, and a bulk density between 0.3 and 1.5 g/cm³, and more preferably between 0.4 and 1.3 g/cm³. The catalyst may be reactivated in a regenerator, using a gas containing molecular oxygen such as oxygen gas or air, alone or in conjunction with water vapour or CO₂. Regeneration of the catalyst is performed by completely or partially removing coke formed on the catalyst by gasifying the coke at high temperatures, preferably at 600-1,000°C.

The hydrogen partial pressure during hydrodealkylation is usually 90 to 5,000 kPa, preferably 180 to 3,000 kPa and more preferably 350 to 2,000 kPa. If the partial pressure of hydrogen is less than about 90 kPa, coke formation on the catalyst is accelerated, thereby significantly decreasing catalytic activity and the hydrodealkylation rate. On the other hand, if the partial pressure of hydrogen is greater than about 5,000 kPa, hydrogenating activity is too high and excessively accelerates cracking of the feed oil, increasing the amount of gas produced to reduce the yield of β-methylnaphthalene.

The reaction temperature is specified to be 450 to 650°C since hydrodealkylation does not proceed sufficiently at temperatures lower than 450°C, resulting in reduction of yields of β-methylnaphthalene, and since hydrodealkylation proceeds excessively and undesirable reactions are accelerated at temperatures higher than 650°C, also resulting in reduction of yields of β-methylnaphthalene.

The contact time is 3 and 35 seconds. If the contact time is shorter than 3 seconds, hydrodealkylation does not proceed sufficiently, resulting in reduction of yields of β-methylnaphthalene, and that if the contact time is longer than 35 seconds, hydrodealkylation proceed excessively and undesirable side reactions are accelerated, also resulting in reduction of yields of β-methylnaphthalene.

According to preferred aspects of the invention, the contact time is 5 to 30 seconds and the reaction temperature is 500 to 630°C. By limiting the reaction conditions to these ranges, undesirable side reactions can be restricted to a controllable extent and thus, yields of β-methylnaphthalene can be enhanced.

The present invention is described below in more detail by way of example only, with reference to the accompanying drawings, in which
Fig. 1 is a schematic representation of apparatus for use in carrying out the method of the invention;
Fig. 2A and 2B are gas chromatograms of a feed oil used in the Examples and the product oil of Example 1, respectively.

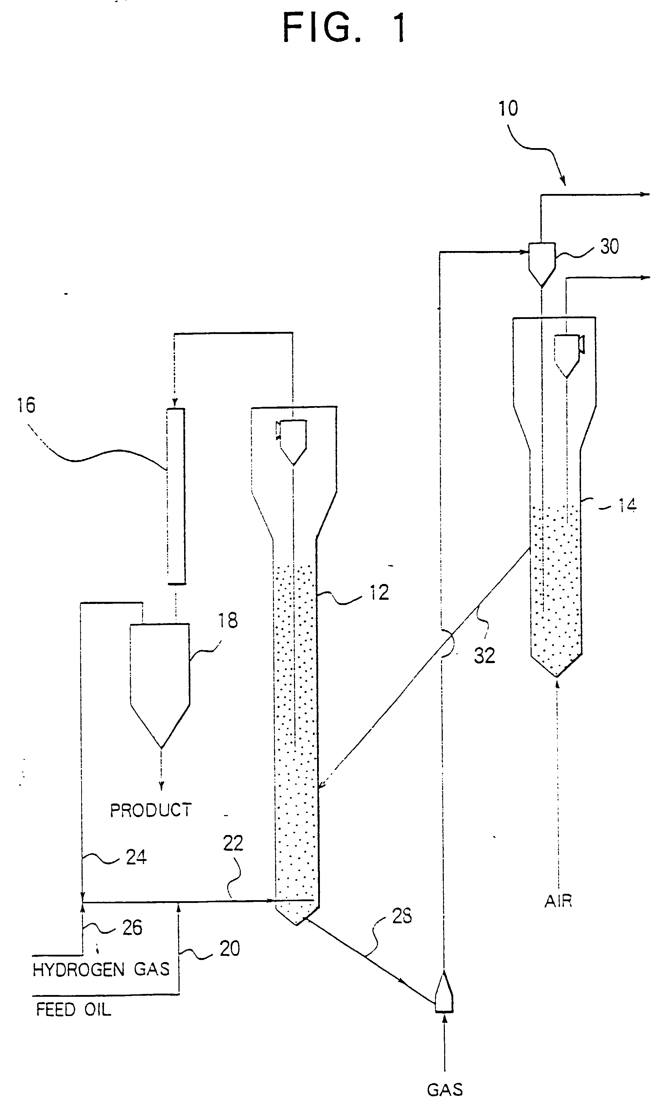

Referring to Fig 1 of the drawings, the apparatus 10 for the reaction is provided with a hollow cylindrical reactor 12 for hydrodealkylation of a feed oil, a hollow cylindrical regenerator 14 for catalyst whose activity has been diminished, a condenser 16 for the product from the reactor 12, and a receiver 18 for the condensed product and non-condensable gas from the condenser 16. A fluidized bed of catalyst is formed in both reactor 12 and the regenerator 14.

The product in the receiver 18 can be easily separated into β-methylnaphthalene and other products by standard distillation.

The equipment making up the apparatus 10 has the following dimensions:

### Reactor 12:

| | |
|---|---|
| Internal diameter: | 8 cm |
| Height: | 4.1 m |
| Amount of catalyst charge: | 7.5 kg |
| Position of the supply port for feed oil: | 20 cm above the bottom |

### Regenerator 14:

| | |
|---|---|
| Internal diameter: | 8 cm |
| Height: | 4.4 m |
| Amount of catalyst charge: | 7.5 kg |

The feed oil is fed through lines 20 and 22 to the lower section of the reactor 12, after being mixed with hydrogen gas. In the reactor 12, the catalyst forms a fluidized bed, having been fluidized by hydrogen gas. The feed oil undergoes hydrodealkylation in the presence of the catalyst to yield a gas containing β-methylnaphthalene. The gas thus leaves the top of the reactor 12 and enters the condenser 16, together with unreacted hydrogen gas. In the condenser 16, the gas is condensed into the product oil except hydrogen gas. The hydrogen gas and the condensed product oil pass to the receiver 18, where the hydrogen gas is separated. The separated hydrogen gas passes through line 24, and reenters the reactor 12, after joining with make-up hydrogen gas passing through line 26.

In the meantime, the catalyst having reduced activity due to carbon deposits leaves the bottom of the reactor 12 and enters the regenerator 14 via line 28 and cyclone 30 by a gas flow transfer operation. In the regenerator 14, the carbon on the catalyst is burned off by air sent through the bottom of the regenerator 14. The regenerated catalyst is recycled to the reactor via line 32.

### Examples 1-10:

Using the apparatus described above, known catalysts A to D having the compositions shown in Table 1, and LCOs having properties shown in Table 2 as a feed oil, catalytic hydrodealkylations were carried out under a variety of conditions shown in Tables 3 and 4.

**Table 1**

| Catalyst | A | B | C | D |
|---|---|---|---|---|
| Active component | V | V | Cr | Ni |
| Content of active component (wt%) | 1.97 | 2.08 | 3.15 | 2.69 |
| Porosity (ml/g) | 0.47 | 0.59 | 0.90 | 0.90 |
| Specific surface area (m²/g) | 263 | 215 | 239 | 239 |
| Weight-mean diameter (µm) | 63 | 61 | 70 | 70 |
| Bulk density (g/ml) | 0.71 | 0.65 | 0.48 | 0.48 |
| Carrier | - Alumina carrier containing silica (10%) - | | | |
| Note: The active components also include oxides of the indicated metals. | | | | |

**Table 2**

| Composition of LCO | Feed oil 1 | Feed oil 2 | Feed oil 2 |
|---|---|---|---|
| Naphthalene (wt.%) | 2.4 | 1.9 | 2.1 |
| β-MN (wt.%) | 6.4 | 5.7 | 6.4 |
| α-MN (wt.%) | 3.2 | 3.0 | 3.3 |
| DMN (wt.%) | 11.9 | 13.6 | 12.7 |
| TMN (wt.%) | 6.0 | 9.6 | 5.6 |
| Others (wt.%) | 70.1 | 66.2 | 69.9 |
| Sulphur (wt.%) | 0.3 | 0.06 | 0.06 |
| Nitrogen (wt.%) | 230 | 260 | 220 |
| Note: α-MN : α-methylnaphthalene β-MN : β-methylnaphthalene DMN: Dimethylnaphthalene TMN: Trimethylnaphthalene | | | |

These abbreviations are also used in Tables 3 and 4.

**Table 3**

| Example No. | 1 | 2 | 3 | 4 | 5 | 6 |
|---|---|---|---|---|---|---|
| Catalyst | A | A | A | B | A | A |
| feed oil | 2 | 2 | 2 | 3 | 2 | 2 |
| Reaction temperature (°C) | 600 | 570 | 530 | 630 | 500 | 480 |
| Contact time (sec) | 15.2 | 12.7 | 16.1 | 6.1 | 28.5 | 29.0 |
| Hydrogen partial pressure (kPa) | 745 | 765 | 765 | 765 | 765 | 765 |

| Composition of Product Oil | | | | | | |
|---|---|---|---|---|---|---|
| Naphthalene (wt.%) | 15.6 | 10.6 | 7.6 | 19.8 | 12.8 | 10.5 |
| β-MN (wt.%) | 9.2 | 10.9 | 11.1 | 7.7 | 11.3 | 9.8 |
| α-MN (wt.%) | 4.2 | 5.2 | 5.3 | 3.7 | 5.3 | 4.8 |
| DMN (wt.%) | 4.1 | 7.9 | 9.5 | 2.6 | 9.1 | 10.4 |
| TMN (wt.%) | 0.4 | 1.2 | 1.2 | 0.3 | 1.1 | 1.6 |
| Others (wt.%) | 22.4 | 27.4 | 39.3 | 22.3 | 41.0 | 36.2 |
| β-MN formation ratio (wt.%) | 25.6 | 28.4 | 26.7 | 25.7 | 27.9 | 19.1 |
| β-MN production ratio (wt.%) | 161 | 191 | 194 | 120 | 198 | 171 |
| Quality evaluation of product oil | G | G | G | G | G | FG |
| Overall evaluation of product oil | G | G | G | FG | G | FG |

**Table 4**

| Example No. | 7* | 8 | 9 | 10* | 11 | 12* | 13* |
|---|---|---|---|---|---|---|---|
| Catalyst | A | C | D | A | A | A | A |
| feed oil | 3 | 1 | 1 | 2 | 2 | 2 | 2 |
| Reaction temperature (°C) | 670 | 600 | 550 | 600 | 530 | 500 | 430 |
| Contact time (sec) | 7.1 | 7.1 | 12.9 | 36.3 | 4.3 | 2.3 | 30.5 |
| Hydrogen partial pressure (kPa) | 686 | 765 | 765 | 765 | 755 | 696 | 765 |

| Composition of Product Oil | | | | | | | |
|---|---|---|---|---|---|---|---|
| Naphthalene (wt.%) | 27.4 | 17.8 | 13.5 | 26.3 | 4.1 | 3.5 | 3.2 |
| β-MN (wt.%) | 2.5 | 7.9 | 9.3 | 3.4 | 8.6 | 6.2 | 6.1 |
| α-MN (wt.%) | 0.9 | 3.8 | 4.4 | 1.7 | 4.0 | 3.3 | 3.1 |
| DMN (wt.%) | 0.4 | 3.3 | 5.0 | 0.6 | 11.6 | 12.0 | 13.2 |
| TMN (wt.%) | 0.0 | 0.3 | 0.6 | 0.0 | 7.5 | 8.1 | 8.1 |
| Others (wt.%) | 13.7 | 22.3 | 27.6 | 18.2 | 41.4 | 45.7 | 47.4 |
| β-MN formation ratio (wt.%) | 7.3 | 25.4 | 28.3 | 9.2 | 13.7 | 3.0 | 3.0 |
| β-MN production ratio (wt.%) | 39 | 123 | 145 | 59 | 150 | 108 | 107 |
| Quality evaluation of product oil | G | G | G | G | G | NG | NG |
| Overall evaluation of product oil | NG | FG | FG | NG | G | NG | NG |

| | | | | | | | |
|---|---|---|---|---|---|---|---|
| * Comparative Examples | | | | | | | |

The composition analyses of the product oils are shown in Tables 3 and 4 under the heading "Composition of Product Oil". The constitution of each composition (wt.%) was based on the following equation:$\text{Wt.% of component X = {(wt. of component X} \text{contained in the product oil produced from} \text{1 wt unit of feed oil) / (wt. of 1 wt. unit} \text{of feed oil)} x 100}$

In Tables 3 and 4, the β-MN formation ratio (represented by %) denotes the conversion ratio of alkylnaphthalene having two or more methyl groups into β-methylnaphthalene, and the β-MN production ratio (represented by %) denotes the ratio based on the following equation:$\text{β-MN production ratio = {(wt of β-MN} \text{contained in the product oil produced from} \text{1 wt unit of feed oil) / (wt of β-MN} \text{contained in 1 wt unit of feed oil)} x 100}$

Accordingly, it can be seen from Tables 3 and 4 that those conditions providing higher β-MN production ratios are more preferable from the viewpoint of product yield.

In Tables 3 and 4, the quality of the product oils was evaluated as "G" where a product oil provides a significantly high purity of β-methylnaphthalene when β-methylnaphthalene is separated by distillation from the product oil under the same distillation conditions; as "NG" when the purity is low; and as "FG" when the purity is intermediate. The overall evaluation of product oils indicates overall evaluation incorporating both the evaluation of β-MN production ratio and the evaluation of quality of the product oil. The overall evaluation is indicated as G (good), FG (acceptable), and NG (not acceptable).

As shown in Tables 3 and 4, the reaction temperatures in Examples 7 and 13 were over 670°C and 430°C, respectively, and the contact times in Examples 10 and 12 were 36.3 seconds and 2.3 seconds, respectively. Thus these comparative Examples, with either the contact time or the temperature being outside the range required by the present invention, yielded low β-MN production ratios of 39%, 107%, 59% and 108%, respectively. In other words, in these Examples, the amount of β-methylnaphthalene in the product oil produced from 1 wt unit of the feed oil is smaller than or substantially equal to the original amount of β-methylnaphthalene contained in 1 wt unit of the feed oil. This indicates that in these Examples, hydrodealkylation proceeded to a much greater extent than that required for formation of β-methylnaphthalene, resulting in complete dealkylation to form naphthalene.

On the other hand, in the other Examples, the β-MN production ratios were significantly higher than 100%, showing that β-methylnaphthalene was formed in amounts greater than the original amounts in the feed oil. Particularly, in Examples 3, 5, 6, and 11, β-MN production ratios of not less than 150% were obtained. The contact times and reaction temperatures in these Examples were approximately 5 to 30 seconds and approximately 500 to 630°C, respectively.

The composition of the feed oil 2 (boiling range: 240-270°C) is shown in the gas chromatogram of Fig. 2A. From this, it can be seen that the feed oil 2 contained, in addition to β-methylnaphthalene, a multiplicity of components each having a boiling point close to the boiling point of β-methylnaphthalene.

Fig. 2B is a gas chromatogram of the product oil (boiling point range: 240-270°C) obtained in Example 1. In this, the components having boiling points close to that of β-methylnaphthalene are greatly reduced. This demonstrates that separation of β-methylnaphthalene from the product oil in Example 1 is much more feasible than the feed oil and thus β-methylnaphthalene of a high purity is obtainable.

## Claims

1. A method for producing β-methylnaphthalene which comprises hydrodealkylating a feed oil containing an alkyl naphthalene having at least two methyl groups in the presence of a catalyst having at least one metal species selected from vanadium, chromium, nickel, rhodium, platinum, iridium, and compounds thereof as an active component and a carrier therefor containing at least one of alumina and silica as its primary component, under a hydrogen partial pressure 90 to 5,000 kPa, at a temperature of 450 and 650°C, and for a contact time of 3 to 35 seconds.

2. A method according to Claim 1, wherein the contact time is 5 and 30 seconds and the reaction temperature is 500 and 630°C.

3. A method according to Claim 1 or Claim 2, wherein the feed oil contains 10 weight % or more of at least one of dimethylnaphthalene and trimethylnaphthalene.

4. A method according to any one or Claims 1 to 3, wherein the feed oil is light cycle oil or its equivalent, produced in the catalytic cracking of heavy fractions of crude oil and having a boiling point range of 200 to 320°C.

5. A method according to Claim 4, wherein the feed oil has a boiling point range of 230 to 300°C.
